(19) Europäisches Patentamt
European Patent Office
Office européen des brevets

(11) **EP 0 821 241 B1**

(12) **EUROPÄISCHE PATENTSCHRIFT**

(45) Veröffentlichungstag und Bekanntmachung des Hinweises auf die Patenterteilung:
**26.10.2005 Patentblatt 2005/43**

(51) Int Cl.$^7$: **G01R 25/00**, G01R 19/00

(21) Anmeldenummer: **97112436.7**

(22) Anmeldetag: **21.07.1997**

(54) **Lock-In-Verstärker mit wählbarer Phasenselektivität**

Lock-in amplifier with selectable phase selectivity

Amplificateur à verrouillage avec sélectivité de phase sélectionnable

(84) Benannte Vertragsstaaten:
**AT CH DE FR GB IT LI SE**

(30) Priorität: **22.07.1996 DE 19629555**

(43) Veröffentlichungstag der Anmeldung:
**28.01.1998 Patentblatt 1998/05**

(73) Patentinhaber: **Columbus Schleif-und Zerspantechnik Hard-und Software GmbH**
**87634 Obergünzburg (DE)**

(72) Erfinder:
• **Zürl, Konrad, Dr.-Ing.**
**87435 Kempten (DE)**
• **Weiss, Armin, Dipl.-Phys.**
**82467 Garmisch-Partenkirchen (DE)**
• **Heel, Helmut, Ing.-u. Dipl. BW-VWA,**
**87663 Lengenwang (DE)**

(74) Vertreter: **Vossius & Partner**
**Siebertstrasse 4**
**81675 München (DE)**

(56) Entgegenhaltungen:
**EP-A- 0 119 711**    **DE-A- 2 646 184**
**US-A- 5 210 484**

**Beschreibung**

Die Erfindung betrifft einen Lock-In-Verstärker mit einer sich von gebräuchlichen Lock-In-Verstärkern unterscheidenden Phasenselektivität, insbesondere einen Lock-In-Verstärker mit wählbarer bzw. veränderbarer Phasenselektivität.

Lock-In-Verstärker dienen zur selektiven Verstärkung sehr kleiner und gestörter Signale. Dabei wird ausgenutzt, daß bestimmte Kenngrößen des zu messenden Signals, wie Frequenz und Phasenlage gegenüber einem Referenzsignal bekannt sind. Die (im folgenden näher beschriebene) Realisierung dieser Verstärkung, deren wesentlichster Schritt eine Multiplikation von Meßsignal und Referenzsignal ist, führt zu einer Abhängigkeit des Verstärkungsfaktors vom Cosinus der Phasenverschiebung zwischen Meßsignal und Referenzsignal.

Bekannte Lock-In-Verstärker bestehen neben Vorverstärkern, Filtern, usw. im wesentlichen aus einem Mischer, d.h. Multiplizierer, der gemäß den folgenden Gleichungen die Signale des Meßeingangs.

$$S_{Meß} = U_{Meß} \cdot \sin (\omega t) \tag{1}$$

und des Referenzeingangs

$$S_{Ref} = U_{Ref} \cdot \sin (\omega t + \varphi) \tag{2}$$

miteinander phasenrichtig multipliziert:

$$S_{Meß} \cdot S_{Ref} = U_{Meß} \cdot U_{Ref} \cdot \sin(\omega t) \cdot \sin(\omega t + \varphi) =$$

$$= 1/2 \, U_{Meß} \cdot U_{Ref} \cdot (\cos \varphi - \cos(2\omega t + \varphi)), \tag{3}$$

wobei $\varphi$ die Phase des Referenzsignals bezogen auf das Meßsignal ist. Die dabei gesuchte Meßgröße $U_{Meß}$ ist ein konstanter oder im Vergleich zu $\sin(\omega t)$ zeitlich langsam veränderlicher Wert. Die bekannte Amplitude $U_{Ref}$ des Referenzsignals ist hier zeitlich konstant.

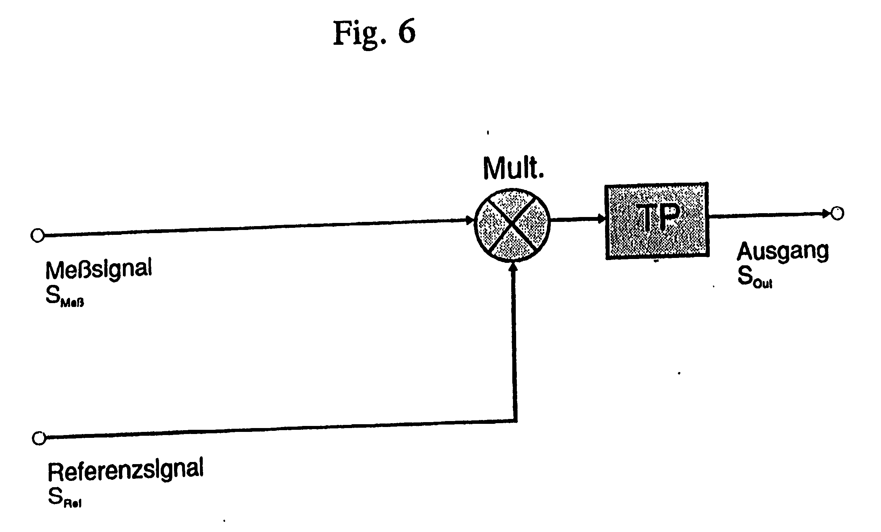

Liegt, wie hier dargestellt, eine Übereinstimmung der Frequenzen von Meßsignal und Referenzsignal vor, so enthält das ermittelte Gesamtsignal nach dem Mischvorgang eine nicht vom $\omega t$ abhängige, d.h. zeitlich konstante, oder nur langsam veränderliche Komponente. Anschließend wird durch einen Tiefpaßfilter TP, vgl. Fig. 6, dessen Grenzfrequenz im allgemeinen variabel ist, nur diese Komponente herausgefiltert. Das Ausgangssignal wird damit zu:

$$S_{Out} = 1/2 \, U_{Meß} \cdot U_{Ref} \cdot \cos \varphi \tag{4}$$

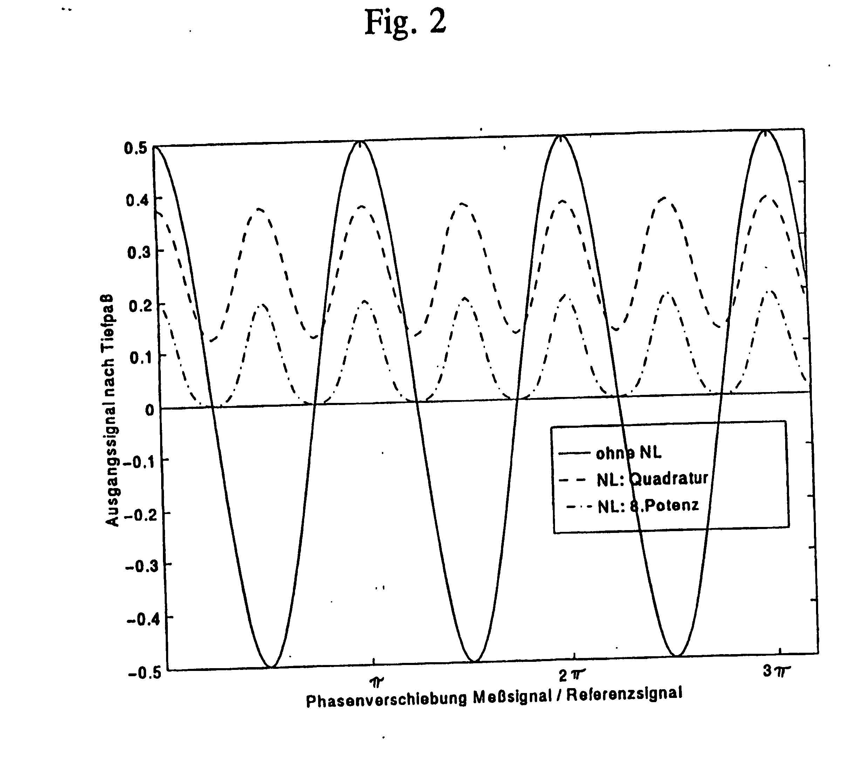

Das Ausgangssignal ist also proportional zur gesuchten Größe $U_{Meß}$ und zeigt eine cosinusförmige Abhängigkeit von der Phasenverschiebung zwischen Meßsignal und Referenzsignal. Diese Abhängigkeit wird in Fig. 2 anhand der durchgezogenen Kurve veranschaulicht.

Herkömmliche Lock-In-Verstärker benutzen am Referenzeingang des Mischers oft statt eines Sinussignals ein Rechtecksignal gleicher Frequenz. Das Referenzsignal stellt in diesen Fällen also eine Überlagerung der Grundschwingung mit ungeraden Harmonischen dar. Damit haben neben der Grundschwingung auch ungerade Harmonische des Meßsignals, d.h. Verzerrungen, einen Anteil an der Bildung des Ausgangssignals. Als weitere Eigenschaft bieten Lock-In-Verstärker oft die Möglichkeit, am Referenzeingang unter Ausschaltung der Grundschwingung nur Harmonische zu verwenden. Dadurch können gezielt harmonische Anteile im Meßsignal betrachtet werden. Die interne Erzeugung des nötigen Referenzeingangssignals für den Multiplizierer erfolgt üblicherweise durch einen PLL- (Phase Locked Loop)-Schaltkreis, d.h. einen Phasenregelkreis, der ein präzises, mit dem externen Referenzsignal phasenstarr gekoppeltes Rechtecksignal ausgibt. Durch diese Eigenschaft ist die Möglichkeit gegeben, zusätzlich zu Beiträgen der Grundschwingung oder unter völliger Nichtbeachtung der Grundschwingung Beiträge von Harmonischen an der Bildung des Ausgangssignals zu betrachten.

Wie bereits angedeutet, zeigt das Ausgangssignal eines bekannten Lock-In-Verstärkers eine cosinusförmige Abhängigkeit von der Phasenverschiebung zwischen Meßsignal und Referenzsignal. Für zahlreiche Anwendungen ist jedoch ein anderer Verlauf dieser Phasenabhängigkeit wünschenswert. Dies kann z.B. für photoakustische Messungen an nicht-gasförmigen Proben zutreffen, bei denen eine selektive Messung von Signalen, die in bestimmten Tiefenschichten der untersuchten Probe erzeugt wurden, entweder in verstärkten oder in verringertem Maße gewünscht wird.

Bei der photoakustischen Spektroskopie tritt ein in einer tieferliegenden Schicht einer nicht-gasförmigen Probe erzeugtes periodisches thermisches Signal aufgrund der Laufzeit der entsprechenden Wärmewelle zeitlich verzögert an der Probenoberfläche auf. Dieses Signal ist also gegenüber dem anregenden periodischen Lichtsignal phasenverschoben. Die phasenselektive Detektion des erzeugten akustischen Signals durch einen Lock-In-Verstärker führt nun zu einer unterschiedlichen Gewichtung von Signalen, die aus verschieden tiefen Schichten der Probe stammen. Bei einem herkömmlichen Lock-In-Verstärker kann jedoch diese Gewichtung nicht beeinflußt, also z.B. selektiv auf bestimmte Tiefenschichten der untersuchten Probe abgestimmt werden. Eine Änderung der Phasenselektivität bezüglich der Grundschwingung ist nicht möglich.

EP 0 119 711 A1 beschreibt einen Lock-In-Verstärker, bei dem vor den Eingang des Multiplizierers ein Frequenzfilter mit optimierter Flankencharakteristik geschaltet ist, so daß in den Signalweg eine Nichtlinearität in der Frequenzdomäne eingebracht wird. Das Referenzsignal wird dabei mit einer definierten Phasenverschiebung beaufschlagt.

US 5 210 484 A beschreibt einen weiteren Lock-In-Verstärker, bei dem das Eingangssignal mit einer Filterung beaufschlagt werden kann.

Der Erfindung liegt die Aufgabe zugrunde, einen Lock-In-Verstärker mit veränderbarer bzw. wählbarer Phasenselektivität bereitzustellen. Diese Aufgabe wird mit den Merkmalen der Ansprüche gelöst.

Bei der Lösung geht die Erfindung von dem Grundgedanken aus, zur Veränderung der Phasenselektivität von Lock-In-Verstärkern in die Signalwege des Meßeingangs und des Referenzeingangs Einrichtungen zur nichtlinearen Transformation der Signale einzufügen.

Diese eingefügten Transformationseinrichtungen, d.h. Nichtlinearitäten können z.B. durch digitale Signalprozessoren oder aber auch durch analoge Bauteile mit nichtlinearer Kennlinie verwirklicht werden. Die verwendeten Nichtlinearitäten für beide Signalwege können mathematisch durch identische Formeln schieben werden. Weiterhin können Transformationseinrichtungen eingesetzt werden, die einerseits eine Quadrierung oder eine höhere Potenzierung (z.B. $x^2$, $x^3$, ...) des jeweiligen Eingangssignals, oder aber andererseits die Potenzierung einer Konstante mit dem jeweiligen Eingangssignal (z.B. $e^x$, $10^x$, ...) bewirken.

Weiterhin können die nichtlinearen Komponenten derart sein, daß die jeweilige nichtlineare Transformation aus mehreren voreingestellten Möglichkeiten wählbar ist.

Die Erfindung wird nachstehend mit Bezug auf die Zeichnungen näher erläutert. Es zeigen:

Fig. 1    die prinzipielle Funktionsweise eines erfindungsgemäßen Lock-In-Verstärkers mit Transformationseinrichtungen in beiden Signalwegen,

Fig. 2    den Verlauf der Ausgangssignale $S_{Out}$ in Abhängigkeit von der Phasenverschiebung $\varphi$ zwischen Meßsignal und Referenzsignal für den bekannten Lock-In-Verstärker und eine erste und zweite Ausführungsform des erfindungsgemäßen Lock-In-Verstärkers,

Fig. 3    den Verlauf der Gewichtung in Abhängigkeit von der Gewebetiefe bei Anwendung eines bekannten Lock-In-Verstärkers zur nicht-invasiven photoakustischen Messung,

Fig. 4    den Verlauf der Gewichtung in Abhängigkeit von der Gewebetiefe bei Anwendung einer ersten Ausführungsform des erfindungsgemäßen Lock-In-Verstärkers zur nicht-invasiven photoakustischen Messung,

Fig. 5    den Verlauf der Gewichtung in Abhängigkeit von der Gewebetiefe bei Anwendung einer zweiten Ausführungsform des erfindungsgemäßen Lock-In-Verstärkers zur nicht-invasiven photoakustischen Messung und

Fig. 6    die prinzipielle Funktionsweise eines bekannten Lock-In-Verstärkers,

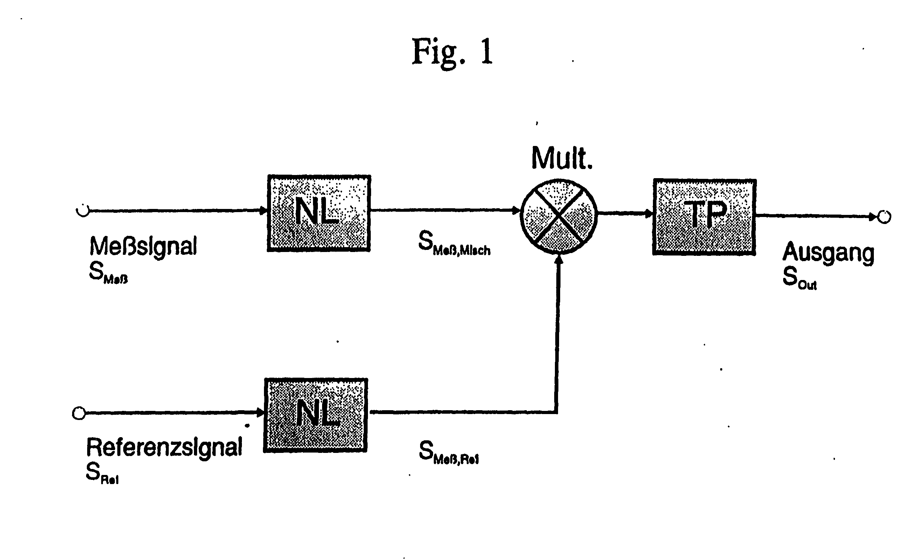

Eine Veränderung der Phasenselektivität eines Lock-In-Verstärkers wird erfindungsgemäß dadurch erreicht, daß in beide Signalwege (Meßeingang und Referenzeingang) nicht-lineare Komponenten eingefügt werden. Fig. 1 zeigt dies am Beispiel einer einfachen Schaltungsvariante eines Lock-In-Verstärkers. Das Meßsignal $S_{Meß}$ und das Referenzsignal $S_{Ref}$ durchlaufen jeweils einen Baustein (NL), der eine nicht-lineare Übertragungsfunktion aufweist. Diese nicht-linearen Komponenten können z.B. durch analoge Bausteine mit nicht-linearer Kennlinie oder durch digitale Signalprozessoren realisiert werden. Die am Ausgang dieser Bausteine anliegenden Signale $S_{Meß,Misch}$ und $S_{Ref,Misch}$ dienen als Eingangssignale für den anschließenden Multiplizierer (Mult.). Nach Durchlaufen eines Tiefpasses (TP) liegt das Signal $S_{Out}$ am Ausgang des Lock-In-Verstärkers an.

In einer ersten erfindungsgemäßen Ausführungsform sind die Nichtlinearitäten im Meßsignalweg und im Referenzsignalweg identisch und bewirken eine Quadrierung des jeweiligen Signals. An den beiden Eingängen des Mischers liegen dann fol= gende Signale an:

$$S_{Meß,Misch} = S_{Meß}^2 = 1/2\, U_{Meß}^2\, (1 - \cos(2\omega t)) \qquad (5)$$

$$S_{Ref,Misch} = S_{Ref}{}^2 = 1/2 \ U_{Ref}{}^2 \ (1 - \cos(2\omega t + 2\varphi)) \tag{6}$$

Das am Ausgang des Mischers anliegende Signal ist dann gegeben durch

$$S_{Meß,Misch} \cdot S_{Ref,Misch} =$$

$$= 1/4 \ U_{Meß}{}^2 \cdot U_{Ref}{}^2 \cdot (1 - \cos(2\omega t) - \cos(2\omega t + 2\varphi)$$

$$+ 1/2\cos 2\varphi + 1/2\cos(4\omega t + 2\varphi)) \tag{7}$$

Nach anschließender Tiefpaßfilterung ergibt sich damit das am Ausgang des Lock-In-Verstärkers anliegende Signal $S_{Out}$ zu

$$S_{Out} = 1/4 \ U_{Meß}{}^2 \cdot U_{Ref}{}^2 \cdot (1 + 1/2\cos 2\varphi) \tag{8}$$

Die Phasenabhängigkeit des Ausgangssignales unterscheidet sich von der eines mit einem herkömmlichen Lock-In-Verstärker, d.h. ohne Transformationseinrichtungen in den Signalwegen erhaltenen Ausgangssignals. Eine Periodizität mit halber Periodenbreite, d.h. proportional zu $\cos 2\varphi$, aber auch mit geringerer Modulationstiefe ist die Folge einer Quadrierung der beiden Signale gemäß der ersten Ausführungsform (gestrichelte Kurve in Fig. 2).
In einer zweiten Ausführungsform (strichpunktierte Kurve in Fig. 2) sollen die im Meßsignalweg und im Referenzsignalweg eingefügten Nichtlinearitäten die achte Potenz des jeweiligen Signales erzeugen. Somit liegen an den beiden Eingängen des Mischers die Signale $S_{Meß,Misch}$ und $S_{Ref,Misch}$ gemäß der folgenden Gleichungen (9) und (10) an:

$$S_{Meß,Misch} = S_{Meß}{}^8 = U_{Meß}{}^8 \cdot 2^{-7} \cdot (35 - 56\cos(2\omega t) + 28\cos(4\omega t)$$

$$- 8\cos(6\omega t) + \cos(8\omega t)) \tag{9}$$

$$S_{Ref,Misch} = S_{Ref}{}^8 = U_{Ref}{}^8 \cdot 2^{-7} \cdot (35 - 56\cos(2\omega t + 2\varphi)$$

$$+ 28\cos(4\omega t + 4\varphi) - 8\cos(6\omega t + 6\varphi) + \cos(8\omega t + 8\varphi)) \tag{10}$$

Das vom Mischer ausgegebene Signal

$$S_{Meß,Misch} \cdot S_{Ref,Misch} \tag{11}$$

wird nach der Tiefpaßfilterung zu

$$S_{Out} = U_{Meß}{}^8 \cdot U_{Ref}{}^8 \cdot 2^{-15} \cdot (2 \cdot 35^2 + 56^2 \cos^2 \varphi + 28^2 \cos 4\varphi$$

$$+ 8^2 \cos 6\varphi + \cos 8\varphi) \tag{12}$$

Fig. 2 zeigt einen Vergleich des Verlaufs der Ausgangssignale $S_{Out}$ nach der Tiefpaßfilterung in Abhängigkeit von der Phasenverschiebung zwischen dem Meßsignal und dem Referenzsignal zwischen einem herkömmlichen Lock-In-Verstärker (durchgezogene Kurve) und einem erfindungsgemäßen Lock-In-Verstärker gemäß der ersten Ausführungsform (gestrichelte Kurve) und der zweiten Ausführungsform (strichpunktierte Kurve). Für die Amplituden des Meßsignals und des Referenzsignals wird dabei $U_{Meß} = 1$ und $U_{Ref} = 1$ angenommen. Wie in Fig. 2 deutlich zu erkennen ist, zeigen die Ausgangssignale eines Lock-In-Verstärkers mit Nichtlinearitäten eine Periodizität mit halber Periodenbreite und verringerter Modulationstiefe gegenüber einem herkömmlichen Lock-In-Verstärker ohne Nichtlinearitäten. Ein wesentlicher Unterschied besteht darin, daß negative Ausgangssignale, die ohne Nichtlinearität z.B. für Phasenverschiebungen von 1/4 π bis 3/4 π auftreten, in den beiden Ausführungsformen nicht auftreten. Weiterhin zeigt die Periodizität des Ausgangssignals $S_{Out}$ der zweiten Ausführungsform (8. Potenz) gegenüber dem Ausgangssignalverlauf der ersten Ausführungsform (Quadrierung) schmalere Maxima und breitere, näher bei der 0-Linie liegende Minima.

Wie bereits zu Beginn erläutert, kann der erfindungsgemäße Lock-In-Verstärker vorteilhaft in der photoakustischen Spektroskopie an nicht gasförmigen Proben, die aus verschiedenartigen, übereinanderliegenden Schichten bestehen, eingesetzt werden. Hier ist z.B. die photoakustische Spektroskopie der menschlichen Körperoberfläche zur nichtinvasiven Bestimmung von Gewebebestandteilen zu nennen. Das bei dieser Spektroskopie im Gasraum über der Probe meßbare akustische Signal gibt den zeitlichen Verlauf der an der Probenoberfläche vorliegenden Temperatur wieder. Dieses Signal sei durch Absorption der einfallenden Strahlung in einer bestimmten Tiefenschicht der Probe erzeugt. Es weist, bedingt durch die Laufzeit der Wärmewelle vom Entstehungsort zur Probenoberfläche gegenüber der anregenden Einstrahlung eine Phasenverschiebung auf. Diese Phasenverschiebung ist abhängig von der Tiefe der Gewebeschicht, in der das Signal erzeugt wurde. Durch die prinzipbedingte Phasenselektivität des erfindungsgemäßen Lock-In-Verstärkers ergibt sich daher eine Tiefengewichtung der Signalanteile, die durch eine Veränderung der Phasenselektivität des Lock-In-Verstärkers variiert werden kann. Die Messung kann gezielt auf bestimmte Schichten fokussiert werden. Dazu sind die Nichtlinearitäten NL derart ausgestaltet, daß unter mehreren voreingestellten Möglichkeiten ausgewählt werden kann.

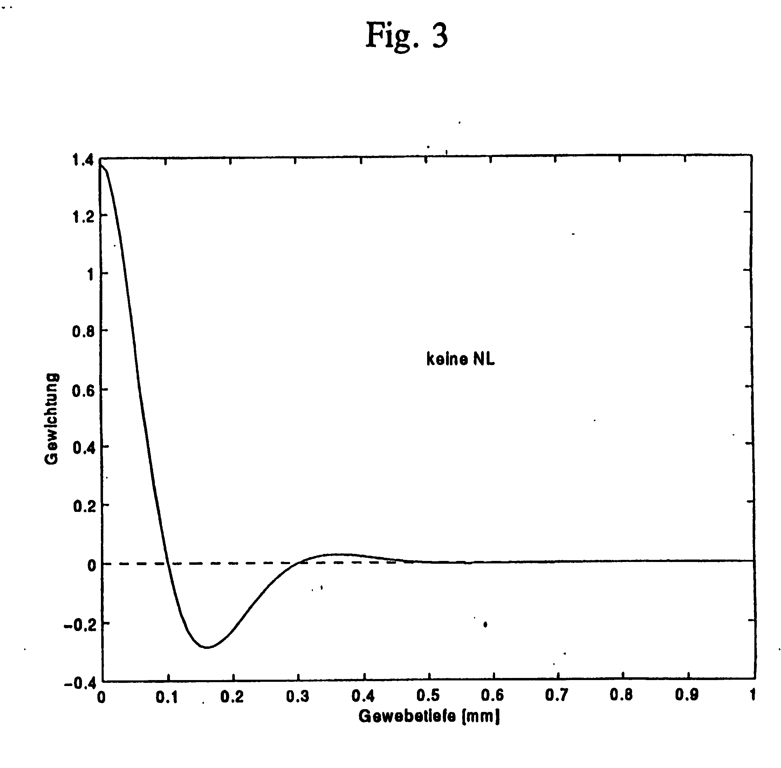

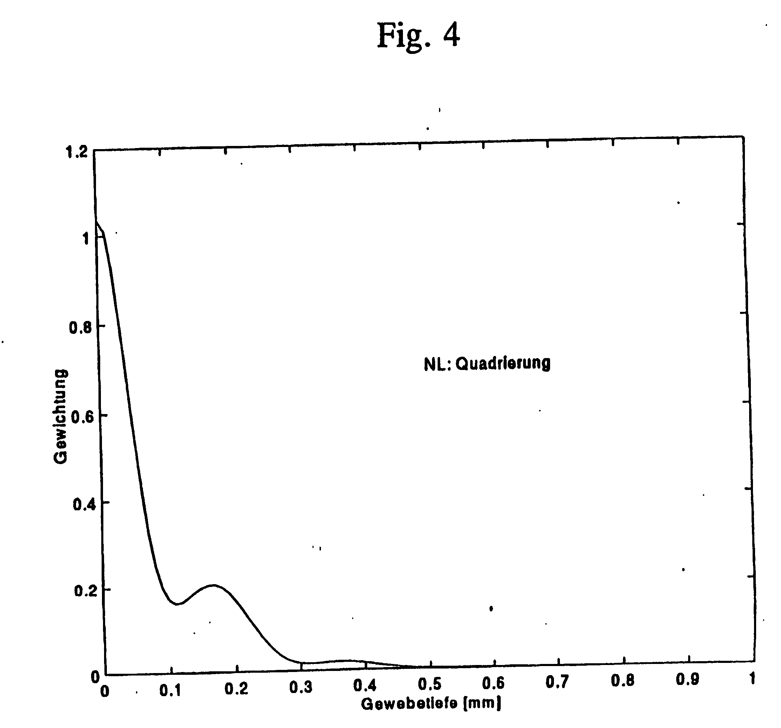

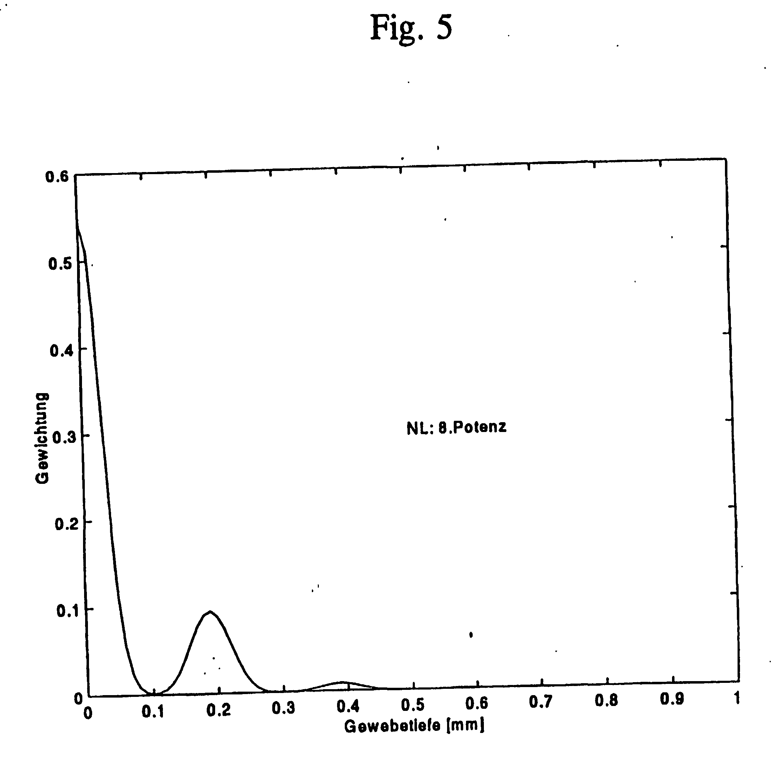

Der vorstehend erläuterte Effekt kann beispielsweise bei der nichtinvasiven photoakustischen Messung von Blutinhaltsstoffen Anwendung finden. Dabei kann durch geeignete Wahl von Tiefengewichtungen die Empfindlichkeit der Messung für Signale, die aus der nichtdurchbluteten obersten Hautschicht, der Epidermis, stammen, minimiert und für Signale aus tieferliegenden, durchbluteten Hautschichten optimiert werden (die Dicke der Epidermis beträgt an geeigneten Stellen ca. 0,1 mm). Figuren 3 bis 5 veranschaulichen für diesen Anwendungsfall und für die beschriebenen Ausführungsformen die Gewichtung der in unterschiedlichen Hauttiefen erzeugten Signale bei der Signalverarbeitung im Lock-In-Verstärker. Die sich für eine Einstrahlung mit einer Lichtwellenlänge von λ = 633 nm ergebende Strahlungsverteilung in der Haut und die Dämpfung der Wärmewelle auf dem Weg von tieferliegenden Gewebeschichten an die Hautoberfläche (zugrundegelegte Modulationsfrequenz der Strahlungsintensität: 5 Hz) sind in den gezeigten Gewichtungskurven bereits berücksichtigt. Wie in Fig. 3 zu sehen ist, treten ohne die Verwendung von Nichtlinearitäten in der Signalverarbeitung positive und negative Gewichtungen von Signalen auf. Für die in diesem Beispiel gewählten Werte von Lichtwellenlänge und Modulationsfrequenz entspricht die Grenze zwischen positiver und negativer Gewichtung etwa der Grenze Epidermis/Dermis. In der ersten (Fig. 4) und zweiten (Fig. 5) Ausführungsform mit geeigneten Nichtlinearitäten treten hingegen nur positive Gewichtungen auf. Dabei sind bei dem Lock-In-Verstärker der zweiten Ausführungsform (8. Potenz) die Gewichtungen stärker auf einzelne Tiefenbereiche konzentriert als bei der ersten Ausführungsform (Quadrierung).

## Patentansprüche

1.   Lock-In-Verstärker mit einer Einrichtung zur nichtlinearen Transformation eines Messsignals und einer Einrichtung zur nichtlinearen Transformation eines Referenzsignals,
      wobei die Transformation für beide Signale mathematisch durch dieselbe identische Formel beschrieben werden kann, und
      wobei entweder

      (a) beide Transformationseinrichtungen eine Quadrierung oder eine höhere Potenzierung des jeweiligen Signals bewirken oder
      (b) beide Transformationseinrichtungen die Potenzierung einer Konstante mit dem jeweilige Signal bewirken, und

      einem Multiplizierer zum Mischen des transformierten Messsignals mit dem transformierten Referenzsignal,
      wobei die Anwendung der Transformationseinrichtungen zu einer wählbaren Phasenselektivität des Verstärkers führt.

2.   Lock-In-Verstärker nach Anspruch 1, wobei mindestens eine Transformationseinrichtung einen digitalen Signalprozessor aufweist.

3.   Lock-In-Verstärker nach Anspruch 1 oder 2, wobei mindestens eine Transformationseinrichtung ein analoges Bauteil mit nichtlinearer Kennlinie aufweist.

4.   Lock-In-Verstärker nach einem der Ansprüche 1 bis 3, wobei die nichtlinearen Transformationen aus mehreren voreingestellten Möglichkeiten wählbar sind.

5. Verwendung des Lock-In-Verstärkers nach einem der Ansprüche 1 bis 4 zur Beeinflussung der Tiefenauflösung bei der photoakustischen Spektroskopie.

6. Verwendung des Lock-In-Verstärkers nach einem der Ansprüche 1 bis 4 zur photoakustischen nichtinvasiven Bestimmung der Konzentration von Substanzen in tierischem oder menschlichem Gewebe.

7. Verwendung des Lock-In-Verstärkers nach einem der Ansprüche 1 bis 4 zur photoakustischen nichtinvasiven Bestimmung der Blutzucker-Konzentration.


**Claims**

1. A lock-in amplifier comprising means for the non-linear transformation of a measuring signal and means for the non-linear transformation of a reference signal,

   wherein the transformation of both signals can be mathematically described by the same identical formula and wherein either

   (a) both transformation means effect a squaring or higher exponentiation of the respective signal
   or
   (b) both transformation means effect the exponentiation of a constant with the respective signal; and

   comprising a multiplier for mixing the transformed measuring signals with the transformed reference signals; wherein the application of said transformation means results in a selectable phase selectivity of the amplifier.

2. The lock-in amplifier according to claim 1, wherein at least one transformation means comprises a digital signal processor.

3. The lock-in amplifier according to claim 1 or 2, wherein at least one transformation means comprises an analogue component with a non-linear characteristic line.

4. The lock-in amplifier according to any of claims 1 to 3, wherein the non-linear transformations are selectable from a plurality of preset options.

5. Use of the lock-in amplifier according to any of claims 1 to 4 for influencing the resolution during photo-acoustic spectroscopy.

6. Use of the lock-in amplifier according to any of claims 1 to 4 for the photo-acoustic non-invasive determination of the substance concentration in animal and human tissue.

7. Use of the lock-in amplifier according to any of claims 1 to 4 for the photo-acoustic non-invasive determination of the sugar concentration in blood.


**Revendications**

1. Amplificateur à verrouillage comprenant :

   - un dispositif pour la transformation non linéaire d'un signal de mesure et un dispositif pour la transformation non linéaire d'un signal de référence,

   dans lesquels la transformation pour les deux signaux peut être décrite mathématiquement par la même formule identique et dans lesquels :

   (a) soit les deux dispositifs de transformation occasionnent une élévation au carré ou une élévation à une puissance plus haute du signal respectif ;
   (b) soit les deux dispositifs de transformation occasionnent l'élévation à une certaine puissance d'une constante avec le signal respectif ; et

- un multiplicateur pour mélanger le signal de mesure transformé et le signal de référence transformé,

l'application des dispositifs de transformation menant à une sélectivité de phase de l'amplificateur, laquelle peut être sélectionnée.

2. Amplificateur à verrouillage selon la revendication 1, dans lequel au moins un dispositif de transformation présente un processeur de signaux numérique.

3. Amplificateur à verrouillage selon la revendication 1 ou 2, dans lequel au moins un dispositif de transformation présente un composant analogique avec une courbe caractéristique non linéaire.

4. Amplificateur à verrouillage selon l'une des revendications 1 à 3, dans lequel les transformations non linéaires peuvent être sélectionnées parmi plusieurs possibilités pré-ajustées.

5. Utilisation de l'amplificateur à verrouillage selon l'une des revendications 1 à 4 pour influencer sur la résolution en profondeur lors de la spectroscopie photo-acoustique.

6. Utilisation de l'amplificateur à verrouillage selon l'une des revendications 1 à 4 pour la détermination photo-acoustique non invasive de la concentration de substance dans le tissu animal ou humain.

7. Utilisation de l'amplificateur à verrouillage selon l'une des revendications 1 à 4 pour la détermination photo-acoustique non invasive de la concentration de sucre dans le sang.

## Fig. 1

Meßsignal
$S_{Meß}$

$S_{Meß,Misch}$

Mult.

$S_{Meß,Ref}$

Ausgang
$S_{Out}$

Referenzsignal
$S_{Ref}$

EP 0 821 241 B1

Fig. 2

# Fig. 3

## Fig. 4

Fig. 5

# Fig. 6

Mult.

TP

Meßsignal
$S_{Meß}$

Referenzsignal
$S_{Ref}$

Ausgang
$S_{Out}$

EP 0 821 241 B1